# NEW EUROPEAN PATENT SPECIFICATION

(11) **EP 2 588 090 B2**
(45) Date of publication and mention of the opposition decision: **22.11.2023**
(45) Mention of the grant of the patent: 19.04.2017
(21) Application number: 11732405.3
(22) Date of filing: 01.07.2011
(51) Int. Cl.: A61K 9/50

(54) **PHARMACEUTICAL ORAL DOSAGE FORMS COMPRISING DABIGATRAN ETEXILATE AND ITS PHARMACEUTICALLY ACCEPTABLE SALTS**
PHARMAZEUTISCHE ORALE DARREICHUNGSFORMEN MIT DABIGATRANETEXILAT SOWIE DESSEN PHARMAZEUTISCH ZULÄSSIGEN SALZEN
FORMES PHARMACEUTIQUES POSOLOGIQUES ORALES COMPRENANT DE L'ÉTÉXILATE DE DABIGATRAN ET SES SELS PHARMACEUTIQUEMENT ACCEPTABLES

(30) Priority: 12.07.2010 SI 201000209; 01.07.2010 SI 201000196
(43) Date of publication of application: 08.05.2013
(73) Proprietor: KRKA, tovarna zdravil, d.d., Novo mesto, 8501 Novo mesto (SI)
(72) Inventor: KROSELJ, Vesna, 1000 Ljubljana (SI); JURSA KSELA, Sonja, 8000 Novo mesto (SI)
(74) Representative: Uexküll & Stolberg
(86) International application number: PCT/EP2011/061140
(87) International publication number: WO 2012/001156

(56) References cited:
- WO-A1-2009/097156
- WO-A1-2010/007016

## Description

### Field of the Invention

The invention relates to processes for the preparation of pharmaceutical oral dosage forms of the active substance ethyl 3-[(2-{[4-(hexyloxycarbonylamino-imino-methyl)-phenylamino]-methyl}-1-methyl-1H-benzimidazole-5-carbonyl)-pyridin-2-yl-amino]-propionate (dabigatran etexilate) and the pharmacologically acceptable salts thereof, in particular dabigatran etexilate methanesulfonate.

### Background of the Invention

Dabigatran is marketed in the form of pellets in hard HPMC capsules under the trade name PRADEXA^{®}. The product contains 75 or 110 mg of dabigatran etexilate in the form of methanesulfonate salt, and the pellets further contain tartaric acid, acacia (gum arabic), hypromellose (HPMC, hydroxypropylmethylcellulose), dimeticone 350 (dimethylpolysiloxane), talc and hydroxypropylcellulose (HPC).

Dabigatran etexilate having the chemical formula (I) 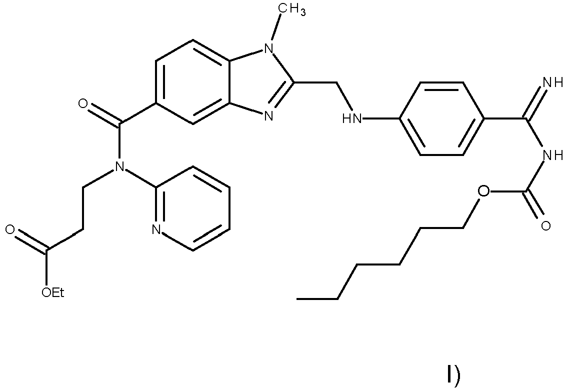 is already known from WO 98/37075, which discloses compounds with a thrombininhibiting effect and the effect of prolonging the thrombin time, under the name 1-methyl-2-[N-[4-(N-n-hexyloxycarbonylamidino)phenyl]-amino-methyl]-benzimidazole-5-yl-carboxylic acid-N-(2-pyridyl)-N-(2-ethoxycarbonylethyl)-amides. The compound of formula (I) is a double prodrug of the compound 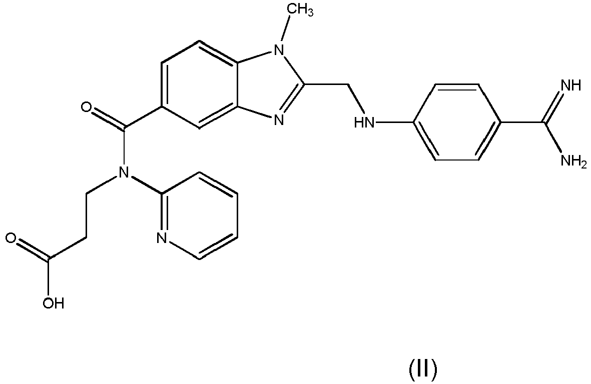 i.e. the compound of formula (I) is only converted into the active compound of formula (II) after entering the body. The main indication for the compound of chemical formula (I) is the post-operative prevention of deep-vein thrombosis. It is expected that it will also be used for the treatment of cardiovascular events in the future.

The solubility of dabigatran, dabigatran etexilate and dabigatran etexilate methanesulfonate in particular, is strongly dependant on the pH value, with increased solubility at acidic pH. On the other hand, it is chemically instable in acidic environment and particularly susceptible to hydrolysis of the pro-drug forming moieties, as well as polymorphic form transformations.

The production of PRADEXA^{®} and the powder layering method according to which it is prepared are described in detail in patent applications WO03/074056, WO2009/118321, WO2009/118322, and WO2010/007016.

WO03/074056 discloses the use of pharmaceutically acceptable organic acids with a water solubility of >1 g/250 ml at 20°C, preferably >1 g/160 ml at 25°C in solid oral preparations, and in particular methanesulphonic acid addition salt of dabigatran etexilate (dabigatran etexilate methanesulphonate). The disclosed pharmaceutically suitable acids are for example tartaric acid, fumaric acid, succinic acid, citric acid, malic acid, glutamic acid and aspartic acid including the hydrates and acidic salts thereof which are applied to the pellets by powder layering. A preferred embodiment of WO03/074056 is a multiparticulate preparation, in particular so-called pellet formulations may be used. The roughly bead-shaped/spherical core region of the pellet consists of a pharmaceutically acceptable organic acid. Then follows the isolating layer, which separates the acid core from the layer containing the active substance. The isolating layer is in turn surrounded by the equally spherically shaped layer of the active substance which may in turn be enclosed in an over-coating which increases the abrasion resistance and shelf life of the pellet.

WO2009/118321 discloses a process for the preparation of approximately spherical/ball-shaped tartaric acid pellets suitable for the manufacture of drug formulations containing active ingredients, the pellets so obtained as such, and the use thereof as a starting material for the production of drug formulations containing active ingredients. The process is characterized in that in a first step the tartaric acid pellets are produced by powder layering, which are sprayed in a second step with an ethanolic isolation suspension which comprises hydroxypropylmethyl cellulose (HPMC).

WO2009/118322 describes a process characterized by a series of partial steps. First, the core is produced from a pharmaceutically acceptable organic acid, preferably tartaric acid by powder layering. The cores are then converted into so-called isolated tartaric acid cores by spraying on an isolating suspension. A dabigatran etexilate suspension prepared subsequently is sprayed onto these coated cores in one or more process steps by means of a coating process. Dabigatran etexilate methanesulfonate, as polymorph I, is suspended together with talc and hydroxypropylcellulose in isopropanol (isopropyl alcohol, 2-propanol, 2-PrOH), the preparation of the suspension being carried out at a temperature not exceeding 30°C. Finally, the active substance pellets thus obtained are packed into suitable capsules.

In WO2010/007016 the process disclosed in WO2009/118322 is further characterized by that polymorph I of dabigatran etexilate methanesulfonate being characterised by a melting point of Tmp. = 180 +- 3°C as determined by DSC at a heating rate: of 10°C/min) is suspended together with talc and hydroxypropylcellulose in isopropyl alcohol, the preparation of the suspension being carried out at a temperature not exceeding 30°C by the circulatory dispersal process.

The formulations described in the above patents and/or patent applications contain a tartaric acid crystal core, which increases the dissolution rate of dabigatran by modifying the pH of the microenvironment when the pellet is dissolved. On the other hand, dabigatran etexilate is unstable in the presence of tartaric acid; therefore, an isolating layer between the tartaric acid core and API (Active Pharmaceutical Ingredient) layer has to be applied. This is particularly tricky since the tartaric acid cores are produced by powder layering of tartaric acid dust onto tartaric acid crystals. The cores produced by such a process are only quasi-spherical and it is very difficult to avoid the formation of satellites which cause defects in the isolating layer. The so-called satellites are small particles adhering to the outside of the otherwise rounded pellets and detracting from the otherwise spherical geometry of the pellets. These lead to impaired storage stability and hence durability of the finished product. Furthermore, protection from humidity during manufacture and storage to prevent hydrolysis of the pro-drug forming moieties and polymorphic form transformations, is recommended. Also, HPMC capsule shells and expensive packaging materials are necessary to assure adequate stability of the final product.

Further on, the technological process described in WO03/074056, WO2009/118321, WO2009/118322, and WO2010/007016 is in all cases powder layering, which is a very complicated and long process, as the tartaric acid crystals of 0.4-0.6 mm size are portionwise and alternately - up to 300 times - sprayed with a solution of acacia and tartaric acid in water, and coated with fine tartaric acid dust with particle size of up to 100 micrometers, preferably below 50 micrometers. It is also complicated to control the particle size of the tartaric acid crystals used as cores as well as the particle size of the tartaric acid dust. The surface of the so called starter pellets (i.e. tartaric acid crystals alternately coated with an aqueous solution of tartaric acid/ acacia (acting as »glue«) and a layer of fine tartaric acid dust) can be rough and have an increased surface area, and in some cases may also not be sufficiently firm for further processing.

WO2005/018615 describes tablets comprising dabigatran etexilate and containing 5 wt. % to 50 wt. % of the active substance (based on the methanesulphonate), 5 wt. % to 50 wt. % of a pharmaceutically acceptable organic acid with a solubility in water of >1 g/250 mL at 20°C as well as other excipients and fillers.

WO2005/023249 describes a pharmaceutical composition comprising: dabigatran etexilate or a pharmaceutically acceptable salt thereof; and a pharmaceutically acceptable lipophilic liquid, solid, or semi-solid carrier system.

Based on the above one can conclude that there is a need for improved solid dosage forms of dabigatran etexilate and its pharmaceutically acceptable salts, preferably dabigatran etexilate methanesulfonate, and/or improved and simplified technological processes for the production thereof.

### Summary of the Invention

The present invention first of all relates to a process for the preparation of a solid oral dosage form comprising dabigatran etexilate or a salt thereof as active substance and comprising a spherical core, wherein
(a) the spherical core is coated with a solution of tartaric acid without a binder and without powder layering of tartaric acid,
(b) the coated core of step (a) is coated with an isolating layer, and
(c) the core coated with an isolating layer of step (b) is coated with further layers wherein at least one of the further layers is a layer comprising the active substance.

It is preferred that the core coated with an isolating layer is coated with a layer comprising the active substance.

The core is preferably comprised of sucrose, microcrystalline cellulose, starch or tartaric acid and preferably is a sugar sphere, and the solid oral dosage form is in particular a pellet.

It is also preferred that the solid oral dosage form comprises less than 20 % by weight of tartaric acid, and that in step (a) a solution of tartaric acid in a mixture of ethanol and water not including a binder is coated on the spherical core.

Further, it is preferred in the present invention that the active substance, i.e. dabigatran etexilate or a salt thereof, such as the methanesulphonate, or a polymorph of these, such as the polymorph I of the methanesulphonate, has a d90 particle size of less than 150µm, more preferably less than 80µm and most preferably less than 40 µm.

The d90 or d50 particle size mentioned herein refers to the size of at least 90 or 50 % by volume of the particles. It is measured by using light scattering methods and in particular using a Malvern Mastersizer.

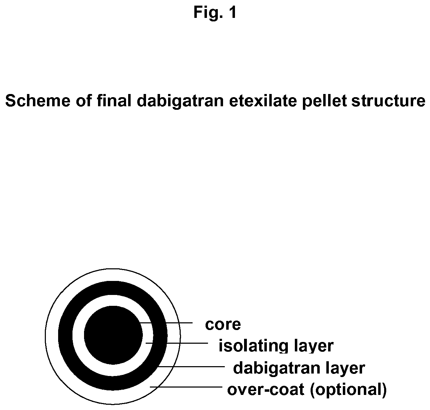

The present invention relates in a preferred embodiment to a process for the preparation of an improved solid oral dosage form of dabigatran etexilate, in particular dabigatran etexilate methanesulfonate. Dabigatran etexilate comprising pellets are prepared by suspension/solution layering of tartaric acid onto spherical neutral or tartaric acid cores without the use of the powder layering method, followed by an isolating layer and active pharmaceutical ingredient layer. Optionally, an overcoat can also be applied to increase the abrasion resistance and shelf life of the final pellets. A scheme of the preferred final dabigatran etexilate pellet structure according to the present invention is shown in Figure 1.

It has surprisingly been found out that dabigatran etexilate, in particular dabigatran etexilate methanesulfonate pellets can also be prepared according to a much simplified and technologically less demanding process than powder layering of tartaric acid crystals with tartaric acid dust and with an aqueous solution of tartaric acid and acacia acting as glue. A commercially available neutral pellet, comprised of sucrose, microcrystalline cellulose or starch, or a commercially available tartaric acid pellet may simply be coated with a solution of tartaric acid, the solution optionally also comprising talc. The dried coated pellets (i.e. starter pellets) obtained by such a process are round, smooth and of narrow size distribution. Their surface is smooth and the coating applied to them is firm. No satellites therefore protrude into the isolating layer and subsequently the layer comprising the active pharmaceutical ingredient thus leading to a stable and bioequivalent pharmaceutical product.

### Detailed Description of the Invention

According to the present invention a preferred improved solid oral dosage form of dabigatran etexilate, in particular dabigatran etexilate methanesulfonate, is produced by suspension/solution layering of tartaric acid onto spherical cores, such as neutral cores comprised of sucrose, microcrystalline cellulose and starch, or tartaric acid pellets, followed by an isolating layer and the layer comprising the active pharmaceutical ingredient. Optionally, an overcoat can be applied.

It has surprisingly been found out that dabigatran etexilate, in particular dabigatran etexilate methanesulfonate pellets can also be prepared according to a much simplified and technologically less demanding process than powder layering of tartaric acid crystals with tartaric acid dust and with an aqueous solution of tartaric acid and acacia acting as glue. A commercially available neutral pellet, comprised of sucrose, microcrystalline cellulose or starch, or a commercially available tartaric acid pellet may simply be coated with a solution of tartaric acid, the solution optionally also comprising talc. The dried coated pellets (i.e. starter pellets) obtained by such a process are round, smooth and of narrow size distribution. Their surface is smooth and the tartaric acid coating as well as the isolating layer applied to them are firm. No satellites protrude into the isolating layer and the layer comprising the active pharmaceutical ingredient thus leading to a stable and bioequivalent pharmaceutical product.

The formulation according to the present invention preferably contains pellets presented in Figure 1. The pellets comprise a neutral core comprised of sucrose, microcrystalline cellulose or starch, or a commercially available tartaric acid pellet; the tartaric acid layer, the isolating layer, the active pharmaceutical ingredient layer and, optionally, an overcoat.

The neutral core comprised of sucrose, microcrystalline cellulose or starch, or a commercially available tartaric acid pellet will be referred to as the »core« in the following.

The core comprising the tartaric acid layer will be referred to as the »starter pellet« in the following.

The pellet comprising the core, the tartaric acid layer and the isolating layer will be referred to as the »isolated pellet« in the following.

The pellet comprising the core, the tartaric acid layer, the isolating layer as well as the active pharmaceutical ingredient layer, and optionally an overcoat, will be referred to as the »final pellet« in the following.

The cores are preferably of round shape and of narrow size distribution. They are selected from but not limited to the group of sugar spheres of different size classes, MCC (microcrystalline cellulose) pellets, tartaric acid pellets etc. Preferably the cores are sugar spheres. Most preferably the cores are sugar spheres of the size 425 - 500 µm.

The starter pellets are in particular prepared from the cores with particle size in the range from 0.2 to 0.8 mm, preferably 0.3 to 0.7 mm and most preferably 0.4 to 0.6 mm as determined by sieving, onto which a solution of tartaric acid is sprayed. The solution is preferably prepared according to the following method: tartaric acid is dissolved in the solvent at room or elevated temperature, preferably at a temperature between 20°C and 80°C. The tartaric acid solution prepared by the method described hereinbefore is then sprayed onto the cores kept moving by rotation. The equipment used is known to the person skilled in the art. In one of the preferred embodiments, a clear solution of tartaric acid without the presence of a binder is prepared in a mixture of ethanol and water.

Spraying is preferably carried out at a defined spray rate and at constant temperature, preferably between 20°C and 80 °C. By the spray rate is meant the amount of tartaric acid solution that is sprayed onto the rotating pellet bed per hour. The spray rate depends on the batch size in the process according to the invention. After spraying is accomplished the starter pellets are dried until a product temperature of about 30°C to 50°C.

The first layer (the tartaric acid layer) comprises tartaric acid and is produced by suspension or solution layering of the cores without powder layering with tartaric acid dust in order to minimize, most preferably avoid the formation of the satellites. Different solvents can be applied for the layering process, such as water or organic solvents, selected but not limited to the group of alcohols (ethyl alcohol, methyl alcohol, 2-propanol, dimethylformamide etc.), acetone and other pharmaceutically acceptable organic solvents. Preferably, alcohols, water and mixtures thereof are used. Most preferably a mixture of ethanol and water is used in the process.

Optionally, other excipients can be added to the solution, such as for example antifoaming agents, or anti-sticking agents.

The content of tartaric acid in the first layer is preferably not lower than 50 %, more preferably not lower than 80 % and most preferably not lower than 90 % w/w. The function of the tartaric acid lies in providing an acidic micro environment when the solid oral dosage form disintegrates to facilitate the dissolving of dabigatran etexilate, preferably dabigatran etexilate methanesulphonate.

The content of tartaric acid in the formulation (capsule content) is usually less than 80 %, preferably less than 60 % and most preferably less than 45 %. Surprisingly it was also found that decreased tartaric acid contents in the final pellet may be applied, which can optionally also be lower than 20% (w/w) without impairing the stability and dissolution of the final pellet.

The second layer is an isolating layer, which is preferably based on a water soluble pharmaceutically acceptable polymer, which can be selected but not limited to the group of cellulose esters (such as hydroxypropyl cellulose, hydroxypropyl methylcellulose, methylcellulose, carboxymethyl cellulose etc.), povidone, acacia, etc. and whose function is to separate the acid sensitive pharmaceutically active ingredient from the tartaric acid layer. Additionally, plasticizers, anticaking agents and coloring agents can be added to the isolating layer. Polyethylene glycols, triethylcitrate, tributylcitrate, triacetine, dibutylsebacate etc. can be used as plasticizers. Talc and silicon dioxide are examples for anticaking agents, preferably talc is used. Optionally dimethylpolysiloxane (dimeticone) or other antifoaming agents can be used to prevent foaming of the solution or suspension. Titanium dioxide, iron oxides and other coloring agents can also be applied.

The isolated pellets are preferably prepared by spraying an isolating suspension/solution onto the starter pellets obtained by the process described hereinabove. To prepare the isolating suspension the selected polymer(s) are in particular dissolved or suspended in the solvent such as water or organic solvents, selected but not limited to the group of alcohols (ethyl alcohol, methyl alcohol, 2-propanol, dimethylformamide etc.), acetone and other pharmaceutically acceptable organic solvents. Preferably, alcohols, water, and mixtures thereof are used. To the solution or suspension of the polymer in the solvent optionally plasticizers, anticaking agents, antifoaming agents and coloring agents are added and the solution or suspension is sprayed onto the starter pellets.

During this continuous process the cores are dried continuously with a supply of air at up to 70°C, preferably from 15°C - 70°C.

The isolated pellets have a uniform and spherical geometry and practically no defects in the isolation caused by satellites. The so-called satellites are small particles adhering to the outside of the otherwise rounded pellets and detracting from the otherwise spherical geometry of the pellets.

The final pellets comprising the pharmaceutically active ingredient are preferably prepared by spraying an active substance suspension onto the isolated pellets. The active substance suspension is prepared using dabigatran etexilate or a pharmaceutically acceptable salt thereof, preferably dabigatran etexilate methanesulfonate in the form of its polymorph I. The polymorph I is characterized inter alia by a melting point of Tmp. = 180 +- 3°C (as determined by DSC; evaluated using peak maximum; heating rate: 10° C/min) and may e.g. be prepared as disclosed in WO 03/074056, Example 3. The targeted production of polymorph I is possible for example using the method described in WO 2005/028468 (particularly Example 1).

In order to prepare the active substance suspension comprising dabigatran etexilate form I in a preferred embodiment for instance isopropyl alcohol (2-PrOH) is adjusted to a temperature range of 10°C - 30°C under a nitrogen atmosphere. Isopropanol is preferably used in anhydrous or almost anhydrous form (99.5 % w/w). If the suspension or solution of dabigatran etexilate methanesulfonate Form I is produced or even stored (aged) at too high a temperature, this may lead to a change in the polymorphic form of the active substance. Particularly preferably the temperature of the manufacturing process is in the range from 0°C to 30°C, particularly preferably between 5°C and 30°C. If another polymorphic form of dabigatran etexilate methanesulfonate or if another salt of dabigatran etexilate is to be used, the solvents to be used are chosen from the group of water or organic solvents, selected but not limited to the group of alcohols (ethyl alcohol, methyl alcohol, 2-propanol, dimethylformamide etc.), acetone and other pharmaceutically acceptable organic solvents and the temperature adapted to that specific salt and/or its polymorphic form.

To the conditioned solvent hydroxypropylcellulose, hydroxypropylmethylcellulose, or another cellulose derivative is preferably added and stirred until dissolved or suspended. Preferably hydroxypropyl cellulose is used. Then the active substance is added and stirring is continued for 15 - 90 minutes, after which optionally one or more anticaking agent, plasticizing agent, antifoaming agent and coloring agent is added. Preferably, talc is added at a constant stirring rate. Stirring is continued after the addition of the last agent for about 15 to 90 minutes. To prepare the final pellet the active substance suspension thus obtained is sprayed onto the isolated pellets as prepared hereinabove.

The final pellets as well as the isolated and/or starter pellets may be prepared by the fluidized bed method comprising the following steps:
i.) preheating of the cores or pellets,
ii.) spraying phase solution or suspension to the cores or pellets, and
iii.) drying of the pellets.

By a fluidized method is meant that the product to be coated has a fluid, preferably air, flowing through it. The material put in is set in motion and kept in motion by this fluid, the nature of the movement being controlled by different equipment-specific inserts. Examples of suitable fluidized bed apparatus are GPCG (= Glatt Particle Coater Granulator), Precision Coater (Aeromatic), Kugelcoater (Hüttlin) or carried out using the Aircoater (Innojet) or Wurster Coater.

Optionally, an overcoat can be applied to the final pellets to increase their abrasion resistance.

It is very important for the stability of the final dosage form, in particular pellets, that the moisture content is kept at a minimum. Preferably, the loss on drying value of the final dosage form should not exceed 2 %, more preferably 1.5 % and most preferably the loss on drying should be less than 1 % (measured on Mettler moisture analyzer, 80°C, 20 min).

Additionally, the loss on drying also of the starter and isolated pellets should be minimal. Preferably the loss on drying value of the starter and isolated pellets should not exceed 2 %, more preferably 1.5 % and most preferably the loss on drying should be less than 1 % (measured on Mettler moisture analyzer, 80°C, 20 min).

The final pellets are usually packed into commercially obtainable capsules such as gelatine or HPMC capsules, or sachets. Alternatively, the simple mixture of isolated pellets and dabigatran is filled into capsules or sachets.

The capsules may be packed into primary packaging having decreased permeability for water vapor such as high density polyethylene (HDPE) containers with closure, such as polypropylene (PP) closure and with or without a desiccant; aluminium foil blisters or polychlorotrifluoroethylene (Aclar^{®}) blisters or any other suitable water vapor low-permeable packaging such as blisters made of multilayer polymeric foil where different polymeric materials are combined into a single foil.

A stabilized pharmaceutical product comprising a package can be produced using a packaging method that utilizes an adsorbent material, such as a molecular sieve, that adsorbs or absorbs moisture in the inner local environment of an impermeable package, so as to prevent hydrolysis and subsequent oxidation products. The adsorbent material can be located in a variety of places. For example, the adsorbent material can be a part of blister foil or can be placed within a porous sachet that, in turn, is located within the sealed package. Naturally, numerous adsorbent materials have applications in a stable pharmaceutical product or a method of the present invention, including a molecular sieve, an activated clay, charcoal, an activated alumina, silica, a zeolite, a bauxite, or any mixture of these materials, to name only a few. An effective amount of the adsorbent material used in a stable pharmaceutical product or in a method of the present invention is that amount sufficient to reduce or eliminate the formation of degradation products. One of ordinary skill can readily determine this amount for a particular embodiment of the present invention using routine laboratory techniques. Moreover, a sealed package of a stable pharmaceutical product or a method of the present invention can be produced from a variety of materials, e.g. metal, glass, plastic, etc. Similarly, the shape of a sealed package can vary. Examples of such shapes include, but certainly are not limited to bottle, a bag, a drum box, and an irregularly shaped container. The sealing of a package of a stable pharmaceutical product or a method of the present invention can be accomplished in a variety of ways. More specifically, heat-sealing, gluing, welding, brazing, mechanical closures, mechanical clamps, or compression can hermetically seal a sealed package of a stable pharmaceutical product of the present invention.

During the packaging and/or storing controlled conditions with low humidity can be used. Additionally inert gases such as nitrogen, argon or helium; or vacuum can be used during the manufacturing of the solid oral dosage forms, the manufacturing of starter, isolated and final pellets as well as during their packaging to assure inert atmosphere around the solid oral dosage form, such as pellet or capsule, in the sealed primary packaging. Inert atmosphere according to present invention means that the concentration of oxygen in the atmosphere around the solid dosage form packed in the primary packaging is less than 10 vol%, preferably less than 5 vol% and most preferably less than 2 vol%. The concentration of oxygen in the atmosphere surrounding the pellets, sachets or capsules can be determined by gas chromatography.

The present invention is further illustrated by the following examples.

### Examples

### A) Preparation of starter pellets (cores with tartaric acid coating)

### Example A8:

| | Material | Quantity per capsule |
|---|---|---|
| 1 | Sugar spheres 425 - 500 µm | 40.00 mg |
| 2 | Tartaric acid | 60.00 mg |
| 3 | Ethanol | q.s. |
| 4 | Purified Water | q.s. |

### Example A9:

| | Material | Quantity per capsule |
|---|---|---|
| 1 | MCC pellets (Cellets ^{®}) | 40.00 mg |
| 2 | Tartaric acid | 60.00 mg |
| 3 | Ethanol | q.s. |
| 4 | Purified Water | q.s. |

### Example A10:

| | Material | Quantity per capsule |
|---|---|---|
| 1 | Tartaric acid pellets (TAP^{®} 400) | 40.00 mg |
| 2 | Tartaric acid | 60.00 mg |
| 3 | Ethanol | q.s. |
| 4 | Purified Water | q.s. |

### Example A11:

| | Material | Quantity per capsule |
|---|---|---|
| 1 | Sugar spheres 425 - 500 µm | 40.00 mg |
| 2 | Tartaric acid | 45.00 mg |
| 3 | Ethanol | q.s. |
| 4 | Purified Water | q.s. |

### Example A12:

| | Material | Quantity per capsule |
|---|---|---|
| 1 | MCC pellets (Cellets ^{®}) | 40.00 mg |
| 2 | Tartaric acid | 45.00 mg |
| 3 | Ethanol | q.s. |
| 4 | Purified Water | q.s. |

### Example A13:

| | Material | Quantity per capsule |
|---|---|---|
| 1 | Tartaric acid pellets (TAP^{®} 400) | 40.00 mg |
| 2 | Tartaric acid | 45.00 mg |
| 3 | Ethanol | q.s. |
| 4 | Purified Water | q.s. |

Preparation of A8-13: Tartaric acid is dissolved in a suitable quantity of purified water and the same quantity of ethanol is added. The resulting solution is sprayed onto starter beads and the obtained pellets are dried until the desired LOD is achieved.

### Example A14:

| | Material | Quantity per capsule |
|---|---|---|
| 1 | Sugar spheres 425 - 500 µm | 40.00 mg |
| 2 | Tartaric acid | 60.00 mg |
| 3 | 2-propanol | q.s. |
| 4 | Purified Water | q.s. |

### Example A15:

| | Material | Quantity per capsule |
|---|---|---|
| 1 | Sugar spheres 425-500 µm | 40.00 mg |
| 2 | Tartaric acid | 45.00 mg |
| 3 | 2-propanol | q.s. |
| 4 | Purified Water | q.s. |

Preparation of A14-15: Tartaric acid is dissolved in a suitable quantity of purified water. 2-propanol is added in an amount sufficient to reduce tackiness of the solution. The obtained solution is sprayed onto cores and the obtained starter pellets are dried until the desired LOD is achieved.

### B) Preparation of Isolated pellets (starter pellets with Isolation coating)

### Example B1:

| | Material | Quantity per capsule |
|---|---|---|
| 1 | Starter pellets A8 | 100.00 mg |
| 2 | HPMC 6cp | 2.23 mg |
| 3 | Dimeticone | 0.04 mg |
| 4 | Talc | 2.23 mg |
| 5 | Ethanol | q.s. |

### Example B2:

| | Material | Quantity per capsule |
|---|---|---|
| 1 | Starter pellets A10 | 100.00 mg |
| 2 | HPMC 6 cp | 2.23 mg |
| 3 | Dimeticone | 0.04 mg |
| 4 | Talc | 2.23 mg |
| 5 | Ethanol | q.s. |

Preparation of B1-2: HPMC is added to ethanol and stirred. When dissolved, dimeticone is added. Talc is suspended in a part of ethanol with ultraturax and added to the HPMC solution. The obtained suspension is sprayed onto the starter pellets.

### Example B3:

| | Material | Quantity per capsule |
|---|---|---|
| 1 | Starter pellets A10 | 100.00 mg |
| 2 | Acacia | 2.23 mg |
| 3 | Dimeticone | 0.04 mg |
| 4 | Talc | 2.23 mg |
| 5 | Purified water | q.s. |

Preparation of B3: Acacia is dissolved in purified water, then dimeticone is added. Talc is suspended in part of purified water and added to the obtained solution. The resulting suspension is sprayed onto the starter pellets.

### Example B4:

| | Material | Quantity per capsule |
|---|---|---|
| 1 | Starter pellets A9 | 100.00 mg |
| 2 | Acacia | 2.23 mg |
| 3 | Talc | 2.23 mg |
| 4 | Purified water | q.s. |

Preparation of B4: Acacia is dissolved in purified water. Talc is suspended in part of purified water and added to the obtained solution. The resulting suspension is sprayed onto the starter pellets.

### C) Final pellets (isolated pellets with active pharmaceutical Ingredient layer)

### Example C1:

| | Material | Quantity per capsule |
|---|---|---|
| 1 | Isolated pellets B1 | 104.50 mg |
| 2 | Dabigatran etexilate mesilate | 86.48 mg |
| 3 | Hydroxypropyl cellulose | 17.30 mg |
| 4 | Talc | 14.72 mg |
| 5 | 2-propanol | q.s. |

Hydroxypropyl cellulose is added to part of 2-propanol and stirred. When dissolved, dabigatran etexilate methanesulphonateis dissolved. Talc is suspended in part of 2-propanol with ultraturax and the suspension is added to the HPC solution). The obtained suspension is sprayed onto isolated pellets B1 and the obtained pellets are dried until appropriate LOD is achieved.

### Example C2:

| | Material | Quantity per capsule |
|---|---|---|
| 1 | Isolated pellets B1 | 144.50 mg |
| 2 | Dabigatran etexilate mesilate | 86.48 mg |
| 3 | Hydroxypropyl cellulose (Klucel EF) | 17.30 mg |
| 4 | Talc | 14.72 mg |
| 5 | 2-propanol | q.s. |

Hydroxypropyl cellulose Klucel EF is added to part of 2-propanol and stirred. When dissolved, milled dabigatran etexilate methanesulphonate methanesulphonate having a d50 particle size of 6.7µm and a d90 particle size of 15.7µm is added. Talc is suspended in part of 2-propanol with ultraturax and the suspension is added to the Klucel solution. The obtained suspension is sprayed onto isolated pellets B1 and the obtained pellets are dried until appropriate LOD is obtained.

### D) Over-coating of final pellets

### Example D1:

| | Material | Quantity per capsule |
|---|---|---|
| 1 | API pellets C1 | 223 mg |
| 2 | HPMC 6 cp | 6.00 mg |
| 3 | Talc | 6.00 mg |
| 4 | Titanium dioxide | 6.00 mg |
| 5 | Ethanol | q.s. |

### Example D2:

| | Material | Quantity per capsule |
|---|---|---|
| 1 | API pellets C1 | 104.50 mg |
| 2 | Povidone K25 | 6.00 mg |
| 3 | Talc | 6.00 mg |
| 4 | Titanium dioxide | 6.00 mg |
| 5 | Ethanol | q.s. |

Preparation of D1-2: HPMC or povidone is added to part of ethanol and mixed. Talc and titanium dioxide are suspended in part of ethanol with ultraturax and mixed to HPMC or povidone solution. The obtained mixture is sprayed onto pellets, which are then dried until the required LOD is achieved.

## Claims

1. Process for the preparation of a solid oral dosage form comprising dabigatran etexilate or a salt thereof as active substance and comprising a spherical core, wherein
(a) the spherical core is coated with a solution of tartaric acid without a binder and without powder layering of tartaric acid,
(b) the coated core of step (a) is coated with an isolating layer, and
(c) the core coated with an isolating layer of step (b) is coated with further layers wherein at least one of the further layers is a layer comprising the active substance.

2. Process according to claim 1, wherein the core coated with an isolating layer is coated with a layer comprising the active substance.

3. Process according to any one of claims 1 or 2, wherein the core is comprised of sucrose, microcrystalline cellulose, starch or tartaric acid and preferably is a sugar sphere.

4. Process according to any one of claims 1 to 3, wherein the solid oral dosage form is a pellet.

5. Process according to any one of claims 1 to 4, wherein the solid oral dosage form comprises less than 20 % by weight of tartaric acid.

6. Process according to any one of claims 1 to 5, wherein in step (a) a solution of tartaric acid in a mixture of ethanol and water not including a binder is coated on the spherical core.

7. A process according to claim 1, wherein said process is for the preparation of final dabigatran pellets, **characterized in that**
(a) a neutral core, chosen from the group of sucrose, microcrystalline cellulose, starch, or a tartaric acid core is coated with a solution of tartaric acid and without a binder and without powder layering with tartaric acid to prepare starter pellets,
(b) the starter pellets as prepared in (a) are coated by spraying an isolating suspension comprising a water soluble pharmaceutically acceptable polymer and optionally plasticizers, anticaking agents, antifoaming and/or coloring agents to prepare isolated pellets, and
(c) the isolated pellets as prepared in (b) are coated by spraying an active substance suspension onto the isolated pellets.

8. A process according to claim 7 **characterized in that** the active substance suspension is prepared using dabigatran etexilate methanesulfonate in the form of its polymorph I.

9. A process according to any of claims 7 or 8, **characterized in that** 2-propanol is used for the preparation of the active substance suspension.

10. A process according to any of claims 7 to 9, **characterized in that** the active substance suspension comprises hydroxypropylcellulose.

## Patentansprüche

1. Verfahren zur Herstellung einer festen oralen Dosierungsform, die Dabigatranetexilat oder ein Salz davon als Wirkstoff enthält und einen kugelförmigen Kern aufweist, bei dem
(a) der kugelförmige Kern mit einer Lösung von Weinsäure ohne ein Bindemittel und ohne Pulverbeschichtung von Weinsäure beschichtet wird,
(b) der beschichtete Kern von Schritt (a) mit einer isolierenden Schicht beschichtet wird, und
(c) der Kern, der mit einer isolierenden Schicht aus Schritt (b) beschichtet ist, mit weiteren Schichten beschichtet wird, wobei mindestens eine der weiteren Schichten eine Schicht ist, die den Wirkstoff enthält.

2. Verfahren nach Anspruch 1, bei dem der mit einer isolierenden Schicht beschichtete Kern mit einer Schicht beschichtet wird, die den Wirkstoff enthält.

3. Verfahren nach einem der Ansprüche 1 oder 2, bei dem der Kern aus Saccharose, mikrokristalliner Cellulose, Stärke oder Weinsäure besteht und bevorzugt eine Zuckerkugel ist.

4. Verfahren nach einem der Ansprüche 1 bis 3, bei dem die feste orale Dosierungsform ein Pellet ist.

5. Verfahren nach einem der Ansprüche 1 bis 4, bei dem die feste orale Dosierungsform weniger als 20 Gew.-% Weinsäure enthält.

6. Verfahren nach einem der Ansprüche 1 bis 5, bei dem in Schritt (a) eine Lösung von Weinsäure in einer Mischung von Ethanol und Wasser, die kein Bindemittel enthält, auf den kugelförmigen Kern aufgeschichtet wird.

7. Verfahren nach Anspruch 1, wobei das Verfahren zur Herstellung der endgültigen Dabigatran-Pellets ist, **dadurch gekennzeichnet, dass**
(a) ein neutraler Kern, der aus der Gruppe von Saccharose, mikrokristalliner Cellulose, Stärke ausgewählt ist, oder ein Weinsäurekern mit einer Lösung von Weinsäure ohne Bindemittel und ohne Pulverbeschichtung mit Weinsäure beschichtet wird, um Ausgangspellets herzustellen,
(b) die wie in (a) hergestellten Ausgangspellets durch Aufsprühen einer isolierenden Suspension beschichtet werden, welche ein wasserlösliches pharmazeutisch annehmbares Polymer und gegebenenfalls Weichmacher, Antibackmittel, Antischaum- und/oder Färbemittel enthält, um isolierte Pellets herzustellen, und
(c) die wie in (b) hergestellten isolierten Pellets beschichtet werden, indem eine Wirkstoffsuspension auf die isolierten Pellets gesprüht wird.

8. Verfahren nach Anspruch 7, **dadurch gekennzeichnet, dass** die Wirkstoffsuspension unter Verwendung von Dabigatranetexilatmethansulfonat in der Form seines Polymorphs I hergestellt wird.

9. Verfahren nach einem der Ansprüche 7 oder 8, **dadurch gekennzeichnet, dass** 2-Propanol zur Herstellung der Wirkstoffsuspension verwendet wird.

10. Verfahren nach einem der Ansprüche 7 bis 9, **dadurch gekennzeichnet, dass** die Wirkstoffsuspension Hydroxypropylcellulose enthält.

## Revendications

1. Procédé pour la fabrication d'une forme pharmaceutique orale solide comprenant comme substance active du dabigatran étexilate ou un sel de celui-ci et comprenant un noyau sphérique, dans lequel
(a) on enrobe le noyau sphérique avec une solution d'acide tartrique sans un liant et sans dépôt en poudre d'acide tartrique,
(b) on enrobe avec une couche isolante le noyau enrobé de l'étape (a), et
(c) on enrobe avec d'autres couches le noyau enrobé avec une couche isolante de l'étape (b), au moins l'une des autres couches étant une couche comprenant la substance active.

2. Procédé selon la revendication 1, dans lequel le noyau enrobé avec une couche isolante est enrobé avec une couche comprenant la substance active.

3. Procédé selon l'une quelconque des revendications 1 et 2, dans lequel le noyau est composé de saccharose, de cellulose microcristalline, d'amidon ou d'acide tartrique et de préférence est une sphère de sucre.

4. Procédé selon l'une quelconque des revendications 1 à 3, dans lequel la forme pharmaceutique orale solide est un granule.

5. Procédé selon l'une quelconque des revendications 1 à 4, dans lequel la forme pharmaceutique orale solide comprend moins de 20 % en poids d'acide tartrique.

6. Procédé selon l'une quelconque des revendications 1 à 5, dans lequel dans l'étape (a) on applique sur le noyau sphérique une solution d'acide tartrique dans un mélange d'éthanol et d'eau n'incluant pas un liant.

7. Procédé selon la revendication 1, ledit procédé étant pour la fabrication de granules finals de dabigatran, **caractérisé en ce que**
(a) on enrobe un noyau neutre, choisi dans l'ensemble constitué par le saccharose, la cellulose microcristalline, l'amidon, ou un noyau d'acide tartrique, avec une solution d'acide tartrique sans un liant et sans dépôt en poudre avec de l'acide tartrique, pour préparer des granules de départ,
(b) on enrobe les granules de départ, tels que préparés en (a), en pulvérisant une suspension isolante comprenant un polymère hydrosoluble pharmaceutiquement acceptable et en option des plastifiants, des agents anti-agglomération, des agents antimousse et/ou colorants, pour préparer des granules isolés, et
(c) on enrobe les granules isolés, tels que préparés en (b), en pulvérisant sur les granules isolés une suspension de substance active.

8. Procédé selon la revendication 7, **caractérisé en ce qu'**on prépare la suspension de substance active en utilisant du méthanesulfonate de dabigatran étexilate sous la forme de son polymorphe I.

9. Procédé selon l'une quelconque des revendications 7 et 8, **caractérisé en ce que** pour la préparation de la suspension de substance active on utilise du 2-propanol.

10. Procédé selon l'une quelconque des revendications 7 à 9, **caractérisé en ce que** la suspension de substance active comprend de l'hydroxypropylcellulose.
